# EUROPEAN PATENT APPLICATION

(11) **EP 3 138 904 A1**
(43) Date of publication of application: **08.03.2017**
(21) Application number: 15183435.5
(22) Date of filing: 02.09.2015
(51) Int. Cl.: C12N 5/071, C08B 15/00, A61L 27/50, C12M 3/00

(54) **DEVICE AND METHOD FOR TISSUE CULTURE COMPRISING A HERMETICALLY SEALED BLOOD CIRCULATION**

(71) Applicant: Bertholdt, Günter, 85253 Erdweg/Kleinberghofen (DE)
(72) Inventor: Bertholdt, Günter, 85253 Erdweg/Kleinberghofen (DE)
(74) Representative: Krauss, Jan

(57) **Abstract**

The present invention relates to the field of tissue engineering and the growth and cultivation of specialized tissues in vitro. More specifically, the present invention provides an endothelial cell culture device module, comprising a) at least one cell culture chamber, b) at least one starter cell culture chamber or tube, c) interconnected with at least one connection opening, wherein said at least one starter cell chamber and said at least connection opening are made of or are lined with bacterial microcrystalline cellulose (BMC), and wherein said at least one starter cell chamber is lined with endothelial cells, and wherein said connection openings are filled with a biocompatible materiel allowing growth factors, like VEGF, to diffuse through it, like collagen or ECM (extracellular material, e.g. Matrigel). The present invention further relates to a cell culture device, comprising said module, and uses of said module and device.

## Description

The present invention relates to the field of tissue engineering and the growth and cultivation of specialized tissues in vitro. More specifically, the present invention provides an endothelial cell culture device module, comprising a) at least one cell culture chamber, b) at least one starter cell culture chamber or tube, c) interconnected with at least one connection opening, wherein said at least one starter cell chamber and said at least connection opening are made of or are lined with bacterial microcrystalline cellulose (BMC), and wherein said at least one starter cell chamber is lined with endothelial cells, and wherein said connection openings are filled with a biocompatible materiel allowing growth factors, like VEGF, to diffuse through it, like collagen or ECM (extracellular material, e.g. Matrigel). The present invention further relates to a cell culture device, comprising said module, and uses of said module and device.

### Background of the invention

Living tissues are characterized by a dense net of blood capillaries which support the cells of the tissue with oxygen and nutrients and are able to remove waste products and toxic substances. The oxygen supply is of particular importance because of oxygen's relatively short diffusion distance of 250-300µm In growing tissues, cells react to oxygen deficiency by emitting signal molecules like VEGF (Vascular Endothelial Growth Factor) which trigger a process in adjacent blood vessels called angiogenesis, which results in an outgrowth of new blood capillaries in the direction of those cells.

It is of great interest to mimic such a process also in vitro since this is the only way to create tissue or organ equivalents with in vivo properties.

Several attempts have been undertaken to find a solution. One method uses existing tissues from animals where the animal cells are removed (decellularization) and then replaced by human cells. By this technique it is possible to remove and replace also the endothelial cells from very small capillaries and to establish a functional new blood perfusion of the tissue. The disadvantages of this method are that the structures are given and cannot be modified which makes each sample unique and standardization difficult - if not impossible. Blood must be pumped in steadily so it must be connected to an external blood supply at all time. (Schanz, J., Pusch, J., Hansmann, J., & Walles, H. Vascularised human tissue models: a new approach for the refinement of biomedical research. J. Biotechnol. 148, 56-63 (2010))

A second system called "Human on a chip" uses small aggregates of cells derived from different organs like liver, kidney, heart etc. called spheroids which are cultivated in small cavities connected to a perfusion system. It is assumed that the single spheroids which form by self-assembly mimic organ properties and in addition allow studying the communication between different organs. The problems with this approach are reproducibility and predictivity since the spheroids are very heterogeneous and only a few organotypic reactions can be detected. The spheroids are an improvement to standard tissue culture but do not truly mimic the complex functionality of a differentiated tissue. (e.g. Materne, E. M., Maschmeyer, I., Lorenz, A. K., Horland, R., Schimek, K. M. S., Busek, M., et al. The Multi-organ Chip - A Microfluidic Platform for Long-term Multi-tissue Coculture. J. Vis. Exp. (98), e52526, doi:10.3791/52526 (2015)).

A third technology uses the possibility to deposit cells precisely on top of each other with a three dimensional printer. This approach allows in principle to reproduce the complexity of a living tissue in vitro. A major problem here is that the building of the tissue with its capillary system requires that the blood supply must be provided almost simultaneously. This challenge allows only making very small constructs which are also very expensive to manufacture because of the equipment needed (Murphy and Atala, 3D bioprinting of tissues and organs Nature Biotechnology 32, 773-785 (2014)).

Moll et al. (in: Moll, C., Reboredo, J., Schwarz, T., Appelt, A., Schürlein, S., Walles, H., Nietzer, S. Tissue Engineering of a Human 3D in vitro. Tumor Test System. J. Vis. Exp. (78), e50460, doi:10.3791/50460 (2013)) disclose a 3D tissue test system that models the *in vivo* situation of malignant peripheral nerve sheath tumors (MPNSTs), which was established with a decellularized porcine jejunal segment derived biological vascularized scaffold (BioVaSc). In the model, a modified BioVaSc matrix was reseeded with primary fibroblasts, microvascular endothelial cells (mvECs) and the S462 tumor cell line. For static culture, the vascular structure of the BioVaSc is removed and the remaining scaffold is cut open on one side (Small Intestinal Submucosa SIS-Muc). The resulting matrix is then fixed between two metal rings (cell crowns). Another option is to culture the cell-seeded SIS-Muc in a flow bioreactor system that exposes the cells to shear stress. Here, the bioreactor is connected to a peristaltic pump in a self-constructed incubator. A computer regulates the arterial oxygen and nutrient supply via parameters such as blood pressure, temperature, and flow rate. This setup allows for a dynamic culture with either pressure-regulated pulsatile or constant flow.

US 6,767,928 discloses a method for generating an extended, osteoconductive mineral coating on a surface of a biomaterial, comprising functionalizing a surface of said biomaterial. US 6,942,830 discloses process for producing a three-dimensional object, comprising providing a three-dimensionally movable dispenser having an outlet opening to dispense a material comprising one or more components. US 6,993,406 discloses a method for forming a three-dimensional, biocompatible, porous scaffold structure using depositing a bio-compatible slurry as discrete elements in a three-dimensional geometry. US 6,730,252 discloses a method for fabricating a filament for use in tissue engineering made of caprolactone.

US 8,492,339 relates to a method of making biomaterials, biodegrable crosslinked urethane-containing polyester (CUPE) elastomers and a scaffold-sheet engineering method for tissue engineering applications

US 8,492,339 relates to controlling the release of growth factors for the promotion of angiogenesis. The growth factors or a polymer matrix are modified by photoactive compounds, such that the growth factors are not released into an active form until they are irradiated with light. The disclosure also relates to tissue engineering scaffolds comprising one or more polymers and at least two growth factors.

There is a great medical need for the replacement of diseased or damaged tissues and to obtain functional test models for drug development. The highly expensive procedures to develop new medicinal therapeutics suffer mainly from the inefficient test systems presently available (animal models and cell culture) which cannot predict reliable the efficacy and safety of a drug candidate in an early phase.

It is the objective of this invention to provide improved means and methods for the cultivation of vascularized mammalian and in particular human tissues in vitro. Other objects and advantages of the present invention will become apparent when studying the description of the present invention as provided herein.

In a first aspect of the present invention, the above object is solved by an endothelial cell culture device module (10), comprising a) at least one cell culture chamber (2), b) at least one starter cell culture chamber or tube (1), c) interconnected with at least one connection opening (3), wherein said at least one starter cell chamber and said at least connection opening are made of or are lined with bacterial microcrystalline cellulose (BMC), wherein said at least one starter cell chamber is lined with endothelial cells, and wherein said connection openings are filled with a biocompatible material allowing growth factors, like VEGF, to diffuse through it, like collagen or extracellular material (ECM, e.g. Matrigel).

The endothelial cell culture device module according to the invention provides the generation and directed growth of capillaries derived from an angiogenetic process into newly formed tissues, which enables the support of multilayered tissues through these new capillaries.

Preferred is the module according to the invention, wherein said at least one connection opening (3) is formed as a connection tube or capillary. Said at least one connection opening (3) can have any suitable diameter or form, depending on the purpose, the tissue to be generated, and the size of the module/device, and preferably has an opening with a diameter of between about 100 to 4000 µm.

In one preferred embodiment of the module according to the invention the cell culture chamber (2) is subdivided with a spacing to match conventional microtiter plate standards that are in particular useful in/for high throughput screening purposes.

In a second aspect of the present invention, the above object is then also solved by a cell culture device, comprising the module according to the invention as described herein, and further comprising at least one oxygenation module (4), and a central tubing module (5), wherein said oxygenation module is connected to least the cell culture chamber (2) and/or the central tubing module (5).

Preferred is the cell culture device according to the invention, wherein said central tubing module (5) is connected to said at least one starter cell culture chamber or tube (1). Further preferred is the cell culture device according to the invention, wherein said oxygenation module (4) comprises a growth chamber where mammalian, and preferably human, lung cells are grown.

In one preferred embodiment of the cell culture device according to the invention, said oxygenation module (4) is covered by an elastic membrane and a fixed top, and an inflatable cushion pump located between said top and the membrane. Preferably, said oxygenation module (4) is connected with the central tubing module (5) and the cell culture chamber (2) in a closed system allowing the device to function autonomously for a longer period of time.

Preferred is the cell culture device according to the invention, wherein said central tubing module (5) comprises one way valves formed similar to the valves in the large veins in mammalian, e.g. human, arms and legs.

Preferred is the cell culture device according to the invention, wherein said device and/or central tubing module (5) is filled with blood, blood substitute and/or other suitable culture medium or mixtures thereof.

Further preferred is the cell culture device according to the invention, wherein said central tubing module (5) comprises at least one inflatable cushion for compressing said tubing in one or more places in order to provide an unidirectional flow of said blood, blood substitute and/or other suitable culture medium or mixtures thereof.

In one preferred embodiment of the module or cell culture device according to the invention, said module or device is formed at least in part by two matching hydrogel layers which are joined and held together by matching solid frames and suitable snap locks. Preferably, said module or device is formed at least in part by 3D-printing and/or laser cutting.

Yet another aspect of the present invention then relates to a method for growing a vascularized tissue, comprising the steps of a) providing the cell culture device according to the present invention as described herein, b) seeding or inoculating the cell culture chamber (2) with cells according to the desired tissue, such as, for example, fibroblast, skin, kidney, pancreatic, liver or lung cells, c) culturing said cells for a suitable period of time, d) optionally, adding a vascularization factor to said cells in said cell culture chamber, and e) harvesting said vascularized tissue by opening at least said module according to the present invention.

Preferred is the method for producing the module according to the invention or the cell culture device according to the invention, comprising forming said module or device at least in part by two matching hydrogel layers which are joined and held together by matching solid frames and suitable snap locks.

Preferred is the method for producing the module according to the invention or the cell culture device according to the invention, wherein said module or device is formed at least in part by 3D-printing and/or laser cutting.

Yet another aspect of the present invention the relates to the use of the module according to the present invention or the cell culture device according to the present invention for at least one of
a) the cultivation of cells and complex tissues up to organ like structures,
b) studying the interaction of blood vessels with other differentiated cells,
c) testing new drug candidates for cells and complex tissues up to organ like structures,
d) testing samples using repeated and complex dosage schemes,
e) the production of large tissues for transplantation e.g. dermis, liver, kidney, heart, muscle, etc.
f) testing the safety and efficacy of drugs on multiple organs simultaneously ("human on a chip"),
g) testing the anti-cancer strategy of anti-angiogenic and anti-vasculogenic therapies, and
h) testing personalized anti-cancer therapies.

The cells to be cultured into e.g. the desired tissue can be any suitable mammalian cells, and are for example commercially available. Cells can be derived from human, monkey, sheep, mouse, rat, pig, goat, horse, cow, and the like, and can comprise, for example, epithelial, heart, fibroblast, skin, kidney, cord blood, muscle, pancreatic, liver, blood, stomach, gut, or lung cells, as well as stem cells. Preferably, the cells in the device are immune-compatible (autologous), i.e. from the same donor, or immune-compatible donors.

In general, the inventive device comprises or consists of a system of interconnecting tubes of various sizes within a transparent hydrogel like material. Because of existing undercuts the system is formed by two matching hydrogel layers (each containing one half of a tube) which are joined and hold together by matching plastic frames and appropriate snap locks. Preferably, the device is enclosed (sealed), in particular hermetically sealed, in order to avoid contaminations, e.g. during storage or transport of the device.

A preferred example of the inventive device consists of or comprises three main parts:
1. A central tubing system containing one way valves like the valves found in the large veins in human arms and legs. The system can be filled with blood, blood substitute or culture medium using the inlet and outlet ports. The system is equipped with an inflatable cushion which compresses the tubing in one or several places resulting in a unidirectional flow. The inner surface of the tubing system is covered completely with endothelial cells and is connected to the two other parts of the device.
2. A tissue incubation area where desired cells can be grown. This area can be subdivided by a grid with a spacing to match conventional microtiter plate standards for high throughput screening purposes. The cells can be covered by a film of culture medium like in standard cell culture. Below the surface there is a dense net of capillaries connected with the central tubing system. Like in the central tubing the capillary system is also lined with endothelial cells. Between the capillaries and the surface of the cell support are holes (Ø 10 - 100 µm) which are filled with collagen or ECM (extra cellular material, e.g. Matrigel). These holes serve as guidance for the formation of new capillaries between the cells on top and the capillaries below. The tissue incubation area also has a connection to the oxygenation part.
3. An oxygenation (gas exchange) area where e.g. human lung cells are grown. This area is organized similar to the incubation area with the same concept of bringing the lung cells in close contact with the blood vessels below. In addition this part is covered by an elastic silicone membrane and a fixed plastic top. Between the plastic cover and the silicone membrane there is an inflatable cushion similar to the one used in the central tubing area. The room between the cells and the silicone membrane contains an air vent in the form of a sterile filter. If the cushion is inflated the silicone membrane will be pushed down and reduce the volume of the chamber below so air will be pressed through the sterile filter. Deflating the cushion will lead to an intake of air from the outside. This process is similar to breathing and has the purpose to exchange CO₂ with O₂ in the blood stream. The oxygenation area is connected with the central tubing system and allows the device to function autonomously for a longer time.

Preferred materials as used are:
i) Suitable are mechanically very stable hydrogels which allow long-term cell attachment and culture (e.g. for more than four weeks). Preferably, the gel is transparent. A particularly preferred hydrogel is the one that is produced by the bacterium *Komagataeibacter xylinus* (former *Gluconacetobacter xylinats*). This material is called bacterial microcrystalline cellulose (BMC). BMC is hydrogel-like but exists only in a semisolid state (no sol/gel transition). It is extremely strong and biocompatible. Up to now no physical-chemical process is known by which a material with similar properties can be produced. BMC is biologically inert and cannot be degraded by mammalian cells. Interestingly cells attach readily to the material, get confluent, and stay differentiated and alive for a long time in contrast to other materials where cells do not survive contact inhibition. Especially endothelial cells stayed alive for more than a year on BMC (own unpublished observation). Another important property of BMC is its high content of water (>99.9%). This has the advantage that two layers of BMC can be joined and pressed together leakage secure and at the same time the material is not weakened or even cut through.
ii) Silicone can be used for molding the two BMC layers to provide oxygen diffusion during the biological production process. Silicone film is also used as a support for the BMC layers so the hydrogel does not dry out and microscopic observation from below is possible. The flexibility of silicone is used as cover of the oxygenation area and also for the two types of inflatable cushions.
iii) Plastic: In general, any suitable plastic can be used for the rigid structures of the devices according to the invention. The plastic as used is preferably suitable for thermoforming and/or injection molding, transparent, and autoclavable. The preferred material for the plastic housing is polycarbonate. It can be autoclaved once without losing quality.
iv) Pumps: The (electrical) pumps to preferably rhythmically inflate and deflate the cushions should be small, attached to the device, and battery driven, so that the device is portable/mobile while functioning.

Surprisingly, the inventive devices allow cells and complex tissues up to organ like structures to be cultivated/grown, while being perfused with blood independently from a living organism. The inventive devices allow for assaying and/or studying the interaction of blood vessels with other differentiated cells.

The device allows the building and maintenance of complex tissues in a reproducible and robust manner and thus facilitates their use as viable system for testing new drug candidates. The long life of the test samples allows the application of repeated and complex dosage schemes. Furthermore, generally all current standard analytical test methods can be applied.

The inventive device can be used in a high throughput environment or system, e.g. when used in screening assays as described herein.

Blood samples can be taken easily, like in animal models and can be compared with metabolic data the cells studied (e.g. the genome, transcriptome, proteome, or antibodies).

Tissues of individual patients can be analyzed, and rare adverse reactions be tested (personalization is easy).

The device can be used to produce large tissues for transplantation that otherwise cannot be produced, e.g. dermis (e.g. for burned or scarred tissue replacement), liver, kidney etc.

Using suitable angiogenic factors and substances (e.g. FGF, VEGF, VEGFR NRP-1, Ang1, Ang2, PDGF, PDGFR, TGF-β, endoglin, TGF-β receptors, MCP-1, Histamine, Integrins, e.g. αVβ3, αVβ5, and α5β1, VE-cadherin, CD31, ephrin, plasminogen activators, and plasminogen activator inhibitor-1, preferably VEGF), a predetermined pattern of capillaries can be created so that the capillaries reach already the surface of the tissue incubation platform. A 3D-printer can then be used to arrange cells in an organ typical spatial order and to create an appropriate capillary connection.

By connecting different organ structures with larger blood vessels the safety and efficacy of drugs can be tested on multiple organs simultaneously ("human on a chip").

The anti-cancer strategy of anti-angiogenic (see, e.g. Yadav L et al. Tumour Angiogenesis and Angiogenic Inhibitors: A Review. J Clin Diagn Res. 2015 Jun; 9(6): XE01-XE05 Epub 2015 Jun 1) and anti-vasculogenic therapies can be studied with the device in detail not possible before.

Individual (e.g. personalized, paediatric) anti-cancer therapies and other therapies can be tested before the actual start of the treatment and the most effective regime can be identified. All features as described herein can be either individually or in any combination thereof be used in order to solve the object of the present invention. The present invention will now be described in more detail in the following non-limiting examples with reference to the accompanying figures. For the purposes of the present invention, all references as cited herein are incorporated by reference in their entireties.

In the Figures,

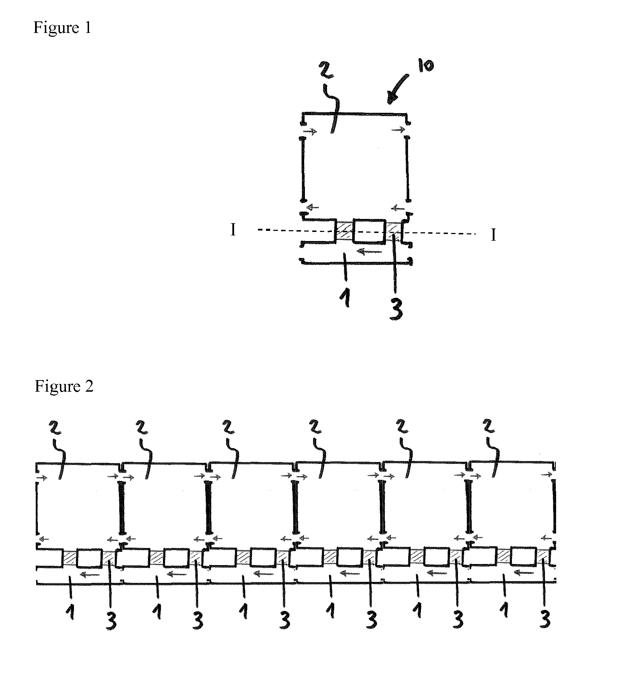

Figure 1 shows a schematic depiction of the endothelial cell culture module (10) according to the invention, with a cell culture chamber (2), with inlets and outlets for medium and oxygen supply (in this case two inlets and two outlets (little arrows), a starter cell culture tube (1) that are interconnected with (in this case) two connection openings (3), herein in the form of a connection tube or capillary. The starter cell chamber and connection openings are made of or are lined with bacterial microcrystalline cellulose (BMC) (not shown), and the starter cell chamber (1) is lined with endothelial cells (not shown). The connection openings (3) are filled with a biocompatible material, like ECM (extra cellular material, e.g. Matrigel).
Figure 2 shows a modular assembly of the endothelial cell culture module (10) of Figure 1.

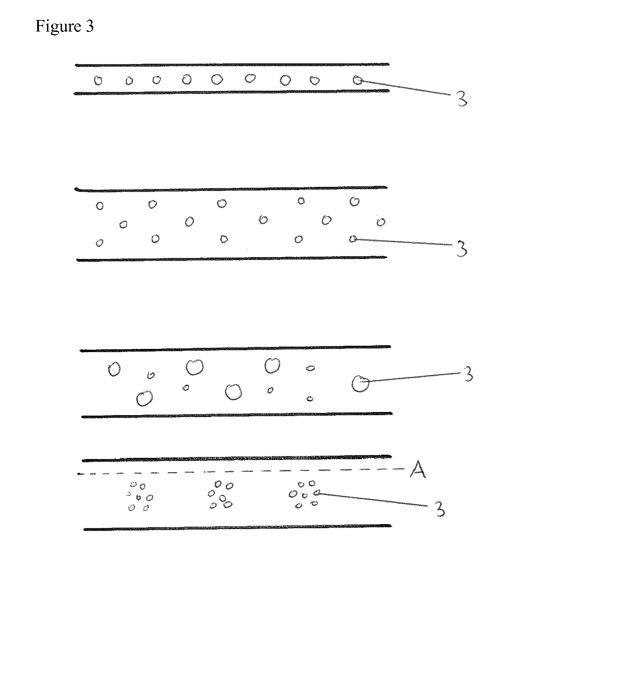

Figure 3 shows cross-sectional views of the module and combinations thereof according to the line I-I of Figure 1. Different arrangements of the connection openings (3) are indicated. It can be seen that the connection openings (3) can be also arranged "custom made", i.e. according to the needs of the culture, and could also be irregular in shape. Furthermore, as an example, the dashed line "A" indicates a position, where the two halves of the module could be connected (i.e. where an upper part and a lower part could be connected.

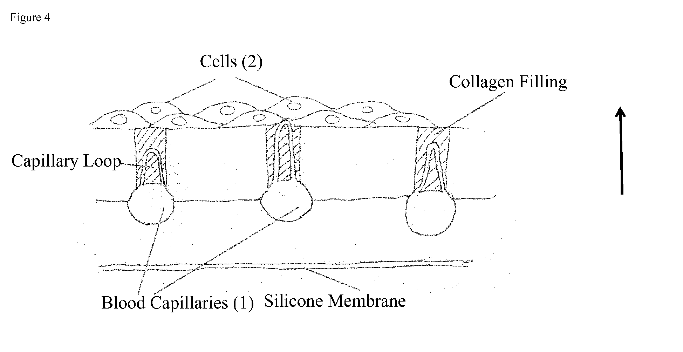

Figure 4 shows a schematic depiction of endothelial cell culture modules according to the invention in a horizontal orientation, with cells in a cell culture chamber (2), starter cell culture tubes ("blood capilliaries", 1) that are interconnected with (in this case) three connection openings (3), herein the form of a capillary with a capillary loop shown. The connection openings (3) are filled with collagen. The arrow indicated the top.

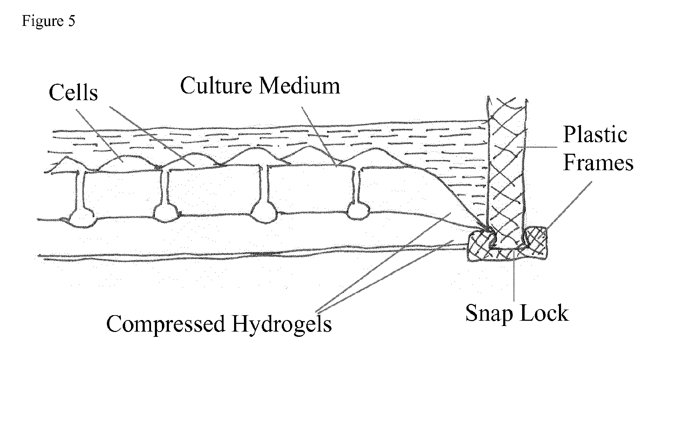

Figure 5 shows a similar view as in Figure 4, with an example for the construction and connection of the device via a snap lock shown.

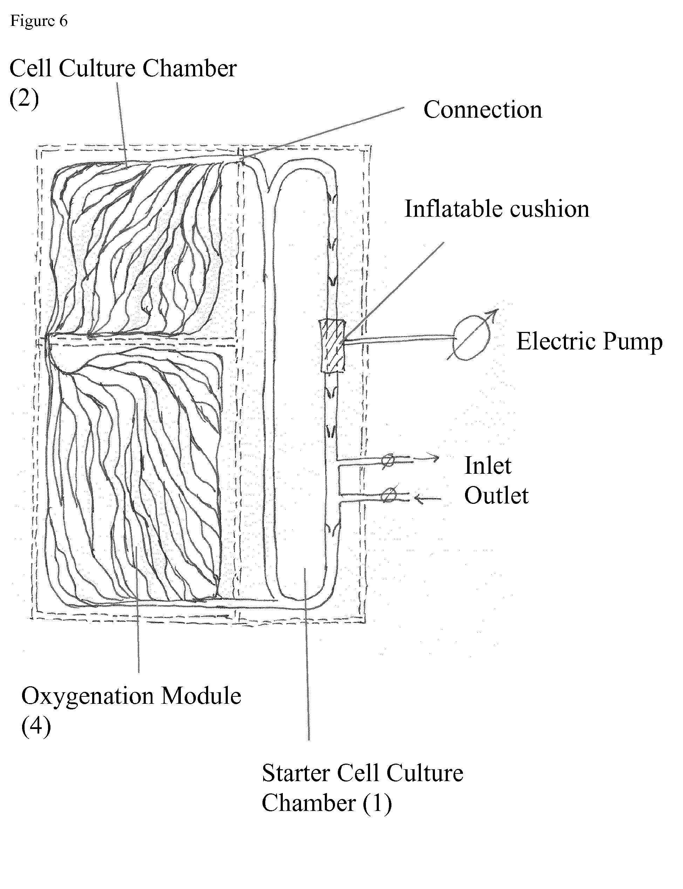

Figure 6 shows a top view of an exemplary cell culture device according to the invention. The device comprises a module according to the invention, and further comprises an oxygenation module (4) and a central tubing module (5), wherein said oxygenation module is connected to least the cell culture chamber (2) and/or the central tubing module (5).

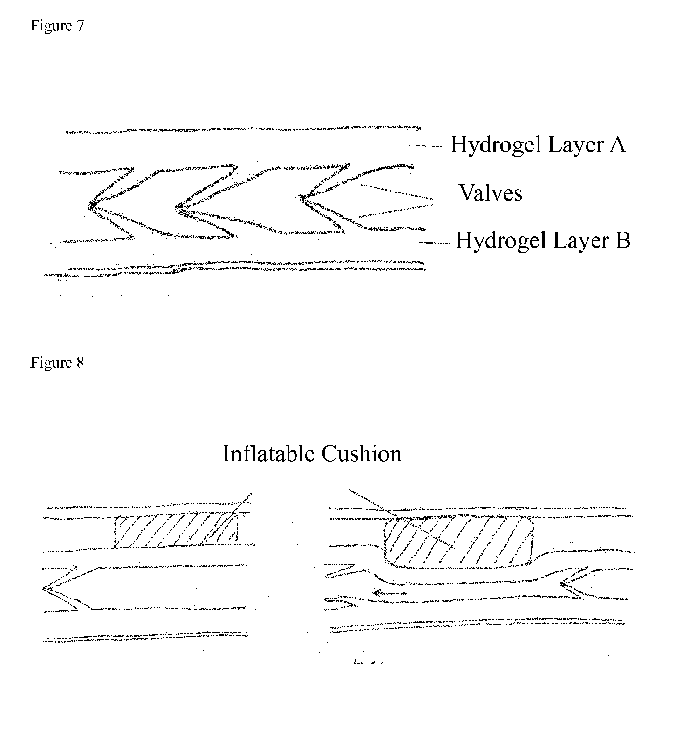

Figure 7 shows the exemplary vein-like valve structure of the tubing structures of the cell culture device according to the invention.
Figure 8 shows the exemplary inflatable cushion pump structure of the cell culture device according to the invention.

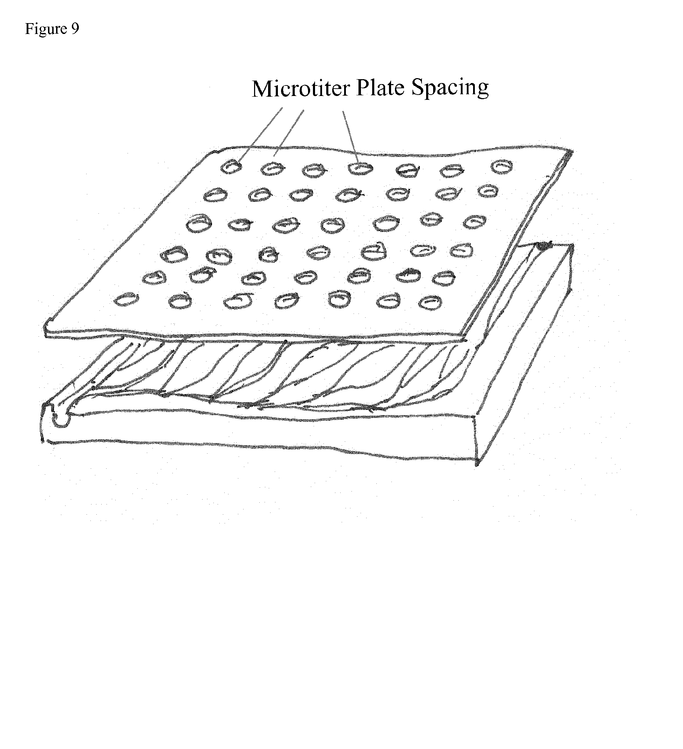

Figure 9 shows a horizontal view of an embodiment of the endothelial cell culture module in a horizontal orientation, with the openings (3) indicated, with the cell culture chamber (2) underneath.

### Examples

### Design and production of the module and the cell culture device according to the invention Building the devices involves both biological (cells) and mechanical procedures.

Two matching hydrogel (BMC) layers are produced using a bacterial cultivation process which requires two bioreactors simultaneously serving as molds. The bioreactors are made of silicone formed by a casting process using molds. There are a variety of options to make these molds. The preferred method uses a wax-3D printer which produces a negative of the structure. The two silicone cushions are produced in a similar way.

The plastic housing consists of 3 parts made of polycarbonate which is manufactured by conventional injection molding. After de-molding the upper BMC-layer is processed further by perforating the incubation and the oxygenation area by a pattern of small holes using a CO₂ laser. Both BMC-layers are treated with hot sodium hydroxide to remove bacterial contaminations and are washed thoroughly with saline.

The device is then assembled within the housing and sterilized by autoclaving. The following procedures are performed under sterile conditions (laminar flow or clean room). The holes in the incubation and oxygenation area are closed by an overlay of liquid collagen or extra cellular matrix components onto the cooled BMC-layer. To prevent a drying out of the hydrogel, it is covered by cell culture medium.

The tubing system (including the cell culture tubes) of the device is filled with a suspension of endothelial cells and culture medium using ports and an electric pump. The cells are allowed to attach and build a homogeneous inner layer throughout the tubing system. The culture medium is then replaced by blood or a blood substitute. The incubation area is inoculated by a suspension of fibroblasts or any other cells desired or a mixture of different cell types.

The oxygenation area as present in this device is preferably inoculated with human lung cells.

Gradients will develop between the cells on top and the underlying endothelial cells with the effect that new capillaries are formed by the endothelial cells into the connection openings that are filled with collagen or ECM-material by angiogenesis. These new capillaries will allow the formation of a new structured multilayered tissue, e.g. on top.

### Details

### 1. Building the molds

The form of tubing system is constructed as a CAD-document and loaded into a high resolution 3Z MAX² Solidscape wax printer (Solidscape Inc, Merrimack, US). The design includes ports to fill the device with a suspension of bacteria. The upper and the lower half are printed separately.

The wax models are placed in matching containers and are overlaid by a two component mixture of silicone and evacuated to remove air bubbles. After the silicone has cured the wax structures are removed mechanically or by heating.

The upper and the lower half were each covered by a flat silicone sheet and pressed together by clamps to avoid leaking.

### 2. Producing the BMC-layers

Bacteria of the strain *Komagataeibacter xylinus* (former *Gluconacetobacter xylinus*) e.g. ATCC 11142 are grown in a medium as described by Hestrin and Schramm (Biochem.J. 1954 Oct.58(2) 345-52) as a standing culture where a pellicle of cellulose is formed. The medium containing the bacteria of several cultures is pooled and the bacteria are concentrated by centrifugation and re-suspension in fresh medium. The end concentration should be about 10¹⁰ bacteria per Milliliter.

The concentrated bacteria suspension is filled into the silicon mold-halves and cultivated for 3-4 days at 30°C. The mold-halves are opened and the solid BMC-halves are carefully extracted.

The BMC-halves are washed thoroughly with hot (80°C) NaOH to remove any bacterial residues. The NaOH is removed by washing with distilled water until reaching neutral pH. Finally the BMC-halves are incubated in physiological saline to establish an osmotic equilibrium to accommodate cell culture.

### 3. Perforation of the upper BMC-halve

The upper part is perforated with holes of a diameter of 50µm using a CO₂ Laser (e.g. LaserPro Spirit GX, Wallburg GmbH, Germany). A preferred distance or spacing between each hole is 250-300µm. The perforations (connection openings) will cover preferably completely the incubation and the oxygenation area.

### 4. Assembling the device

The two complimentary BMC-halves are carefully assembled and fixed by the plastic harness.

### 5. Sterilization of the device

The resulting device is then sterilized by autoclaving at 121°C for 20 Min.

### 6. Closing the perforations (connection openings)

The upper BMC-half is cooled to 4°C and soaked with a Geltrex®hESC (life technologies, Thermo Fisher) solution. Subsequently the temperature is raised to 37°C which leads to a solidification of the Geltrex and a closure of the connection openings.

### 7. Endothelial cell attachment

The device is filled with a suspension of endothelial cells (preferably human umbilical vein or artery derived) at a concentration of 10⁷ cells/ml. The inflatable cushion is assembled to the device and a rhythmically pressure is applied resulting in a unidirectional flow of the culture medium.

In this example, the cell culture chamber is seeded with fibroblast cells at a concentration of 10⁷ cells/ml, and the oxygenation area is seeded with lung cells (e.g. alveolar type II cells) at a concentration of 10⁷ cells/ml.

### 8. Use of the device

The device is filled with blood or blood substitute. Switching on the second electric pump the silicone film on top of the oxygenation area starts moving and exchanges the air between the cells and the silicone film.

It can take up to several weeks until there will be an effective oxygen transfer to the circulating blood or blood substitute. Therefore, the oxygen content of the blood or the blood substitute should be controlled constantly, and, if necessary, blood or the blood substitute has to be replaced at regular intervals.

### Reference numerals

- 1: starter cell culture chamber or tube
- 2: cell culture chamber
- 3: connection opening
- 4: oxygenation module
- 5: central tubing module
- 10: cell culture device module

## Claims

1. An endothelial cell culture device module (10), comprising
a) at least one cell culture chamber (2),
b) at least one starter cell culture chamber or tube (1),
c) interconnected with at least one connection opening (3),
wherein said at least one starter cell chamber and said at least connection opening are made of or are lined with bacterial microcrystalline cellulose (BMC),
wherein said at least one starter cell chamber is lined with endothelial cells, and
wherein said connection openings are filled with a biocompatible material allowing suitable growth factors, like VEGF, to diffuse through it, such as collagen or extracellular material (ECM, e.g. Matrigel).

2. The module according to claim 1, wherein said at least connection opening (3) is formed as a connection tube or capillary.

3. The module according to claim 1 or 2, wherein said at least connection opening (3) has an opening with a diameter of between about 100 to 4000 µm.

4. The module according to any of claims 1 to 3, wherein the cell culture chamber (2) is subdivided with a spacing to match conventional microtiter plate standards for high throughput screening purposes.

5. A cell culture device, comprising the module according to any of claims 1 to 4, and further comprising at least one oxygenation module (4) and a central tubing module (5), wherein said oxygenation module is connected to least the cell culture chamber (2) and/or the central tubing module (5).

6. The cell culture device according to claim 5, wherein said central tubing module (5) is connected to said at least one starter cell culture chamber or tube (1).

7. The cell culture device according to claim 5 or 6, wherein said oxygenation module (4) comprises a growth chamber where lung cells are grown.

8. The cell culture device according to any of claims 5 to 7, wherein said oxygenation module (4) is covered by an elastic membrane and a fixed top, and an inflatable cushion pump located between said top and the membrane.

9. The cell culture device according to any of claims 5 to 8, wherein said oxygenation module (4) is connected with the central tubing module (5) and the cell culture chamber (2) in a closed system allowing the device to function autonomously for a longer period of time.

10. The cell culture device according to any of claims 5 to 9, wherein said central tubing module (5) comprises one way valves formed like valves in the large veins in mammalian arms and legs.

11. The cell culture device according to any of claims 5 to 9, wherein said central tubing module (5) comprises at least one inflatable cushion for compresses said tubing in one or more places in order to provide a unidirectional flow of said blood, blood substitute or suitable culture medium.

12. The module according to any of claims 1 to 4 or the cell culture device according to any of claims 5 to 11, wherein said module or device is formed at least in part by two matching hydrogel layers which are joined and held together by matching solid frames and suitable snap locks.

13. A method for growing a vascularized tissue, comprising the steps of
a) providing the cell culture device according to any of claims 5 to 12,
b) seeding the cell culture chamber (2) with cells according to the desired tissue, such as, for example, fibroblast, skin, kidney, pancreatic, liver or lung cells,
c) cultivating said cells for a suitable period of time,
d) optionally, adding a vascularization factor to said cells in said cell culture chamber, and
e) harvesting said vascularized tissue by opening at least said module according to any of claims 1 to 4.

14. A method for producing the module according to any of claims 1 to 4 or the cell culture device according to any of claims 5 to 12, comprising forming said module or device at least in part by two matching hydrogel layers which are joined and held together by matching solid frames and suitable snap locks.

15. Use of the module according to any of claims 1 to 4 or the cell culture device according to any of claims 5 to 12 for
a) the cultivation of cells and complex tissues up to organ like structures,
b) studying the interaction of blood vessels with other differentiated cells,
c) testing new drug candidates for cells and complex tissues up to organ like structures,
d) testing samples using repeated and complex dosage schemes,
e) the production of large tissues for transplantation e.g. dermis, liver, kidney etc.
f) testing the safety and efficacy of drugs on multiple organs simultaneously ("human on a chip"),
g) testing the anti-cancer strategy of anti-angiogenic and anti-vasculogenic therapies, and
h) testing personalized anti-cancer therapies.
